(19) **Europäisches Patentamt**

**European Patent Office**

**Office européen des brevets**

(11) **EP 1 139 871 B1**

(12) **EUROPÄISCHE PATENTSCHRIFT**

(45) Veröffentlichungstag und Bekanntmachung des Hinweises auf die Patenterteilung:
**27.12.2006 Patentblatt 2006/52**

(51) Int Cl.:
***A61B 5/107*** *(2006.01)*

(21) Anmeldenummer: **99959323.9**

(22) Anmeldetag: **24.11.1999**

(86) Internationale Anmeldenummer:
**PCT/EP1999/009080**

(87) Internationale Veröffentlichungsnummer:
**WO 2000/033736 (15.06.2000 Gazette 2000/24)**

(54) **VORRICHTUNG UND VERFAHREN ZUR UMFANGSBESTIMMUNG**

DEVICE AND METHOD FOR MEASURING THE PERIPHERY OF A BODY

DISPOSITIF ET PROCEDE POUR MESURER LE CONTOUR D'UN CORPS

(84) Benannte Vertragsstaaten:
**AT BE CH CY DE DK ES FI FR GB GR IE IT LI LU MC NL PT SE**

(30) Priorität: **10.12.1998 DE 19857098**

(43) Veröffentlichungstag der Anmeldung:
**10.10.2001 Patentblatt 2001/41**

(73) Patentinhaber: **TQ-Systems GmbH**
**82229 Seefeld (DE)**

(72) Erfinder: **OSER, Daniel**
**D-82237 Wörthsee (DE)**

(56) Entgegenhaltungen:
**DE-A- 3 223 711 DE-U- 29 607 994**
**GB-A- 1 594 833**

**Beschreibung**

Die vorliegende Erfindung betrifft eine Vorrichtung und ein Verfahren zur Bestimmung des Umfangs eines Körpers.

In nahezu allen technischen Bereichen ist es häufig erforderlich, den Umfang eines Körpers zu bestimmen bzw. zu kontrollieren. Im Bereich der Fertigung zählt eine Umfangsmessung oft zum Bestand einer Qualitätskontrolle von Produkten. Dabei ist es oft wichtig, daß diese Umfangsbestimmung möglichst exakt, gleichzeitig aber auch schnell und einfach erfolgt, da es sich zumeist nur um eine, das eigentliche Verfahren begleitende Messung handelt, die den störungsfreien Ablauf des Verfahrens zeitlich nicht sehr beeinträchtigen soll. Eine weitere Anwendung findet ein Umfangsmesser im Bereich der Medizin. Hier ist es beispielsweise im Bereich der venösen Verschlußplethysmographie nötig, den exakten Umfang von beliebigen Extremitäten eines Patienten bestimmen. zu können.

Die DE 3 223 711 beschreibt eine Vorrichtung und ein Verfahren zur Vermessung der unteren Extremitäten, z.B. als Basis für die Anfertigung von medizinischen Kompressionsstrümpfen, wobei die Umfangsmessung mit einer durch elektromotorischen Antrieb in ihrem Umfang veränderlichen Meßbandschlinge erfolgt, die die Extremitäten bei der Aufnahme des Umfangsmaßes mit einem konstanten, elektronisch geregelten Anpreßdruck umschließt. Mit Hilfe eines das Meßbandraster abtastenden elektronischen Augenmoduls und einer Zählerelektrode wird das Meßergebnis digital zur Anzeige gebracht.

Eine Aufgabe der vorliegenden Erfindung ist es, eine Vorrichtung und ein Verfahren bereitzustellen, mit deren Hilfe eine schnelle, einfache und genaue Bestimmung des Umfangs eines Körpers möglich ist.

Erfindungsgemäß wird diese Aufgabe durch die im Anspruch 1 beschriebene Vorrichtung und durch das in den Verfahrensabsorüchen 7 und 8 beschriebene Verfahren gelöst.

Vorteilhafte Weiterbildungen der erfindungsgemäßen Vorrichtung sind in den Ansprüchen 2-6 beschrieben.

Vorzugsweise weist die erfindungsgemäße Vorrichtung eine Einheit zur Berechnung und zur Ausgabe des Umfangs des Körpers aus der Länge $L_D$, dem Weg $W_D$ und einem vorrichtungsabhängigen Geometriefaktor G auf. Dadurch ist eine schnelle und gleichzeitig exakte Bestimmung des Umfangs gewährleistet. Vorzugsweise ist der Wegmesser unmittelbar mit einer Signalverarbeitungseinheit, bevorzugt mit einem Mikroprozessor oder besonders bevorzugt mit einem Computer, gekoppelt, in die der gemessene Weg $W_D$, den der Draht beim Spannen entlang dem Umfang des Körpers durch die Antriebseinrichtung hindurch zurückgelegt hat, eingelesen und direkt ausgewertet werden kann. Die vorbekannten Parameter der Vorrichtung wie die Länge $L_D$ des bekannten Längenabschnitts des Drahtes und der vorrichtungsabhängige Geometriefaktor G können dabei in der Signalverarbeitungseinheit als feste Größen gespeichert und zu jeder Zeit abgerufen werden, bzw. vorzugsweise unmittelbar in ein zur Auswertung dienendes Programm integriert werden. Der resultierende Umfang erscheint sodann vorzugsweise auf einem, direkt mit der Verarbeitungseinheit verbundenen Bildschirm bzw. Display.

In einer anderen bevorzugten Ausführungsform der erfindungsgemäßen Vorrichtung ist die Antriebseinrichtung ein Mangelgetriebe mit einer Antriebsrolle und einer Traktionsrolle. Durch Einbringen des Drahtes zwischen die Antriebsrolle und die Traktionsrolle wird die Vorrichtung in sich geschlossen. Die Antriebseinrichtung, der Draht und der Lagesensor bilden somit ein in sich geschlossenes, vollständiges System zur Bestimmung des durch den Draht umschlungenen Umfangs eines Körpers. Nach Inbetriebsetzen der Antriebsrolle fungiert der Draht als Überträger bzw. Transmitter des Drehmoments der Antriebsrolle auf die Traktionsrolle, so daß die beiden Rollen sich synchron zueinander drehen und dabei gleichzeitig den Draht einziehen. Die Länge, um welche der Draht zwischen den beiden Rollen eingezogen wird, kann simultan mit Hilfe des an die Traktionsrolle gekoppelten Wegmessers zu jedem Zeitpunkt gemessen werden. Sobald der Draht gespannt ist, d.h. sobald er unmittelbar an dem Körper, dessen Umfang zu messen ist, anliegt, übt der Draht eine endliche Zugkraft auf den Lagesensor, mit welchem er in Wirkkontakt steht, aus. Der Lagesensor reagiert auf diese, auf ihn wirkende Zugkraft durch die Erzeugung eines Signals. Dieses Signal ist dabei vorzugsweise wiederum rückgekoppelt mit der Antriebsrolle, die bei Erscheinen des Signals ihren Betrieb einstellt, so daß der Draht nicht weiter von den beiden Rollen, d.h. der Antriebs- und der Traktionsrolle eingezogen wird.

Vorzugsweise wird als Lagesensor ein induktiver Wegaufnehmer verwendet, dessen Impedanz sich in Abhängigkeit von einer, hier durch den Draht, auf ihn wirkenden Kraft ändert. Dabei liegt die Kraft, die nötig ist, damit der Lagesensor anspricht, weit unterhalb der Kompressibilitätsgrenze des Körpers, dessen Umfang zu bestimmen ist. Somit ist sichergestellt, daß der Körper durch den Draht nicht komprimiert wird und somit seinen Umfang ändert, noch bevor der Lagesensor anspricht, d.h. ein Signal gibt. Zur Sicherstellung, daß die Kompressibilitätsgrenze des Körpers bei der Umfangsmessung noch nicht erreicht ist, wird der Draht vorzugsweise nach dem ersten von dem Lagesensor gegebenen Signal noch weiter um einen vorbestimmten Betrag von der Antriebseinrichtung eingezogen. Der Vergleich der am Wegmesser ablesbaren eingezogenen Strecke des Drahtes nach dem ersten von dem Lagesensor gegebenen Signal mit der nach dem ersten Signal simultan aufgetretenen entsprechenden Längenausdehnung des Lagesensors bei weiterem Einzug des Drahtes durch die Antriebseinrichtung bietet eine gute Kontrollmöglichkeit; beide Strecken müssen nämlich identisch sein solange der Körper nicht durch den nun unmittelbar auf dem Umfang des Körpers anliegenden Draht komprimiert wird.

In einer bevorzugten Ausführungsform weist die Antriebseinrichtung einen Elektromotor auf.

In einer bevorzugten Ausführungsform der erfindungsgemäßen Vorrichtung weist mindestens ein Teil der Oberflächen der Antriebseinrichtung, die mit dem Draht während des Spannens entlang dem Umfang des Körpers in Berührung kommen eine durch eine photomechanische oder mechanische oder chemische oder elektrochemische Behandlung erzielbare Aufrauhung auf. Bei der bereits erwähnten bevorzugten Ausgestaltung der Antriebseinrichtung als Mangelgetriebe wird durch solch eine gezielte Aufrauhung der Traktionsrolle eine gute Haftung des Drahtes auf der Traktionsrolle erreicht und somit ein einfacher, kontinuierlicher und stetiger Einzug des Drahtes gewährleistet. Eine gute Haftung zwischen Draht und Traktionsrrolle ist im Falle des Mangelgetriebes auch für dessen einwandfreie Funktionsweise, d.h. für das Zusammenspiel von Traktionsrrolle, Antriebsrolle und Draht unverzichtbar.

Während die Haftung zwischen Draht und Traktionsrolle vergleichsweise groß sein sollte, muß die Haft- und Gleitreibung des Drahtes auf dem zu bestimmenden Umfang des Körpers vergleichsweise klein sein, so daß der Draht beim Spannen auf dem Umfang einwandfrei entlanggleiten kann. Vorzugsweise wird von daher ein glatter Draht gewählt, insbesondere ein Nylondraht. Darüber hinaus wird vorzugsweise ein Material, insbesondere Nylon, für den Draht gewählt, so daß die Querschnittsfläche des Drahtes auch unter Belastung in weiten Grenzen im wesentlichen konstant bleibt. Damit ergibt sich eine konstante Kontaktfläche zwischen dem Draht und der Traktionsrolle und somit konstante Prozeßbedingungen. Vorzugsweise besteht der Draht aus einem biegeweichen Material, um beim Spannen des Drahtes um den zu bestimmenden Umfang eine gute Stoffschlüssigkeit zu gewährleisten. Auch bezüglich dieser Materialeigenschaft eignet sich Nylon als Drahtmaterial gut. Eine weitere bevorzugte Materialeigenschaft für den Draht ist eine hohe Reißfestigkeit. Auch diese Eigenschaft wird von Nylon erfüllt. Insofern besteht der Draht, mit dessen Hilfe der Körper entlang seines Umfangs zumindest teilweise umschlossen wird, in einer bevorzugten Ausführungsform der Erfindung aus Nylon. Nylon ist ein kostengünstiges, gleichzeitig aber auch ein sehr stabiles, insbesondere reißfestes, glattes und biegeweiches Material. Der Draht ist somit in einem weiten Bereich sehr stabil gegenüber auf ihn wirkende Zugkräfte. Bei Legen des Drahtes um den Körper und Spannen des Drahtes durch seinen Einzug mittels des Zusammenspiels von Antriebsrolle und Traktionsrolle, besteht somit keine bzw. nur eine äußerst geringe Gefahr, daß der Draht reißt, bevor die Umfangsbestimmung vorgenommen werden konnte. Vorzugsweise wird ferner ein Drahtmaterial gewählt, dessen Längenänderung in Abhängigkeit von der Temperatur verschwindend klein ist. Auch diese Eigenschaft wird von Nylon zufriedenstellend erfüllt.

In einer weiteren bevorzugten Ausführungsform der Erfindung wird ein hochempfindlicher Sensor verwendet, der bereits bei einer sehr geringen, auf ihn durch den Draht ausgeübte Zugkraft reagiert, d.h. ein Signal gibt. Vorzugsweise wird hierbei, wie bereits erwähnt, ein induktiver Wegaufnehmer verwendet. Somit ist es möglich, mit Hilfe der vorliegenden Erfindung auch den Umfang solcher Körper zu bestimmen, die eine endliche Kompressibilität haben, d.h. nicht starr sind. Mit solchen Körpern hat man es beispielsweise im Falle von Extremitäten zu tun. Im Bereich der Verschluß- und/oder Kompressionsphlethysmographie gilt es, den Umfang von Extremitäten in Abhängigkeit vom Zustand der entsprechenden Gefäße zu bestimmen. Je nach Zustand und Funktion der Gefäße können die Extremitäten in ihrem Umfang variieren. Diese oft kleinen, aber wesentlichen Umfangsveränderungen können mit Hilfe der vorliegenden Erfindung sehr genau, d.h. mit einer Auflösung von etwa 1 $\mu$m, und schnell bestimmt werden.

Ferner bezieht sich die vorliegende Erfindung auf ein entsprechendes Verfahren zur Bestimmung des Umfangs eines Körpers, wobei das Verfahren mindestens die folgenden Schritte aufweist:

a) Legen eines Drahtes mit einem bekannten Längenabschnitt der Länge $L_D$ in Umfangrichtung um den Körper, wobei der Draht mit einem Lagesensor in derartigem Wirkkontakt steht, daß der Lagesensor ein Signal S ausgibt, sobald der Draht auf den Lagesensor mindestens eine vorbestimmte Zugkraft Z ausübt,

b) Einbringen des Drahtes in eine Antriebseinrichtung, die mit einem Wegmesser in Wirkkontakt steht,

c) Spannen des Drahtes entlang des Umfangs des Körpers durch Inbetriebnahme der Antriebseinrichtung bis der mit dem Draht in Wirkkontakt stehende Lagesensor ein Signal gibt,

d) Ablesen des mit dem Wegmesser bestimmten Wegs $W_D$, den der Draht beim Spannen entlang des Umfangs des Körpers durch die Antriebseinrichtung hindurch zurückgelegt hat,

e) Bestimmen des Umfangs des Körpers aus der Länge $L_D$, dem Weg $W_D$ und einem vorrichtungsabhängigen Geometriefaktor G.

Darüber hinaus bezieht sich die vorliegende Erfindung auf die Verwendung der erfindungsgemäßen Vorrichtung und des erfindungsgemäßen Verfahrens im Bereich der Verschluß- und/oder Kompressionsplethysmographie, insbesondere zur Bestimmung des Umfangs von Extremitäten.

Die erfindungsgemäße Vorrichtung und das erfindungsgemäße Verfahren werden nunmehr anhand der folgenden Figur beschrieben. Es zeigt:

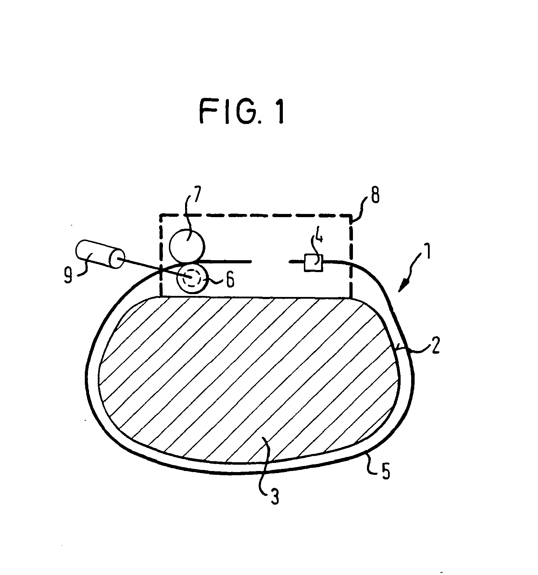

Fig.1 Vereinfachte Darstellung einer erfindungsgemäßen Vorrichtung zur Bestimmung des Umfangs eines Körpers.

Figur 1 zeigt eine vereinfachte Darstellung einer erfindungsgemäßen Vorrichtung 1 zur Bestimmung des Umfangs 2 eines Körpers 3. Zunächst wird die Vorrichtung 1 an einer Stelle des zu messenden Umfangs 2 des Körpers 3 angeordnet. An dem Lagesensor 4 der erfindungsgemäßen Vorrichtung 1 ist ein Draht 5 mit einem bekannten Längeabschnitt der Länge $L_D$ angeordnet, der zur Bestimmung des Umfangs 2 des Körpers 3 in Umfangsrichtung um den Körper 3 herumgelegt wird, d.h. der Draht 5 umschließt, zumindest teilweise, den Körper 3 entlang des zu messenden Umfangs 2. Der Draht 5 wird dann zwischen eine Antriebsrolle 6 und eine Traktionsrrolle 7, die zusammen die Antriebseinheit der Vorrichtung 1 bilden, eingeführt. Die Traktionsrolle 7 ist mit einem Wegmesser gekoppelt. Die Antriebsrolle 6 wird von einem Antrieb 9, vorzugsweise einem Elektromotor angesteuert. Der Lagesensor 4, die Antriebsrolle 6 und die Traktionsrolle 7 zusammen mit dem Wegmesser sind von einem Gehäuse 8 umgeben. Sobald der Draht zwischen den beiden Rollen eingeführt ist, ist die Vorrichtung geschlossen. Durch den Antrieb wird die Antriebsrolle 6 in Rotation versetzt. Über den Draht 5, der hier als Transmitter fungiert, wird das Drehmoment der Antriebsrolle 6 auf die Traktionsrolle 7 übertragen, so daß beide Rollen synchron zueinander rotieren und dabei den Draht 5 mit einziehen. Dadurch wird der Draht 5, der an seinem anderen Ende mit dem Lagesensor 4 verbunden ist, nach und nach gespannt bis er stoffschlüssig an dem Umfang 2 des Körpers 3 anliegt. Sobald der Draht 5 unmittelbar auf dem Körper 3 aufliegt, übt der Draht 5 eine Zugkraft auf den Lagesensor 4 aus, was wiederum bewirkt, daß dieser, sobald die vom Draht ausgeübte Zugkraft einer vorbestimmten Zugkraft entspricht, ein Signal erzeugt. Einhergehend mit dem Signal wird der Betrieb der Antriebsrolle 6 unterbrochen, d.h. der Draht 5 wird nicht weiter gespannt. Mit Hilfe des Wegmessers kann nun genau die Länge $W_D$ des Weges, den der Draht 5 während des Spannens entlang des Umfangs 2 des Körpers 3 durch die beiden Rollen 6, 7 hindurch zurückgelegt hat, abgelesen werden. Mit Kenntnis der gesamten Länge $L_D$ desjenigen Längenabschnitts des Drahtes 5, der der Wegmessung mit Hilfe des Wegmessers zugänglich ist, sowie des vorrichtungsabhängigen Geometriefaktors G läßt sich nun sehr schnell und einfach auf den Umfang 2 des Körpers 3 rückrechnen:

$$L_D - W_D + G = \text{Umfang 2.}$$

**Patentansprüche**

1. Vorrichtung (1) zur Bestimmung des Umfangs (2) eines Körpers (3), wobei die Vorrichtung (1) mindestens die folgenden Elemente aufweist:

   a) einen Draht (5) mit einem bekannten Längenabschnitt mit der Länge $L_D$,
   b) einen Lagesensor (4), der in derartigem Wirkkontakt mit dem Draht (5) steht, daß er ein Signal S ausgibt, sobald der Draht (5) auf den Lagesensor (4) mindestens eine vorbestimmte Zugkraft Z ausübt.
   c) eine Antriebseinrichtung (6, 7) zum Spannen des Längenabschnitts der Länge $L_D$ des Drahts (5) entlang des Umfangs (2) des Körpers (3), wobei die Antriebseinrichtung (6, 7) mit dem Lagesensor (4) derart verbunden ist, daß bei Ausgabe des Signals S das Spannen des Drahtes (5) durch die Antriebsrichtung (6, 7) gestoppt werden kann,
   d) einen mit der Antriebseinrichtung (6, 7) in Wirkkontakt stehenden Wegmesser zur Messung des Wegs $W_D$ den der Draht (5) beim Spannen entlang des Umfangs (2) des Körpers (3) durch die Antriebseinrichtung (6, 7) hindurch zurücklegt,
   e) so daß der Umfang (2) des Körpers (3) aus der Länge $L_D$, dem Weg $W_D$ und einem vorrichtungsabhängigen Geometriefaktor G bestimmt werden kann,

   und wobei die Vorrichtung **dadurch gekennzeichnet ist, daß** des Draht (5) ein loses Ende hat und die Antriebseinrichtung (6,7) zum Einziehen des losen Endes des Drahtes (5) geeignet ist.

2. Vorrichtung nach Anspruch 1, **dadurch gekennzeichnet, daß** die Vorrichtung eine Einheit zur Berechnung und Ausgabe des Umfangs (2) des Körpers (3) aus der Länge $L_D$, dem Weg $W_D$ und einem vorrichtungsabhängigen Geometriefaktor G aufweist.

3. Vorrichtung nach einem der vorhergehenden Ansprüche, **dadurch gekennzeichnet, daß** die Antriebseinrichtung (6, 7) ein Mangelgetriebe mit einer Antriebsrolle (6) und einer Traktionsrolle (7) ist.

4. Vorrichtung nach einem der vorhergehenden Ansprüche, **dadurch gekennzeichnet, daß** mindestens ein Teil der Oberflächen der Antriebseinrichtung (6, 7), die mit dem Draht (5) während dessen Spannens entlang dem Umfang (2) des Körpers (3) in Berührung kommen, eine durch eine Laserbehandlung erzielbare Aufrauhung aufweisen.

5. Vorrichtung nach einem der vorhergehenden Ansprüche, **dadurch gekennzeichnet, daß** der Draht (5) ein Nylondraht ist.

6. Vorrichtung nach einem der vorhergehenden Ansprüche, **dadurch gekennzeichnet, daß** die Antriebseinrichtung (6, 7) einen Elektromotor aufweist.

7. Verfahren zur Bestimmung des Umfangs (2) eines Körpers (3), das mindestens die folgenden Schritte aufweist:

a) Legen eines Drahtes (5) mit einem Losen Ende mit einem bekannten Längenabschnitt der Länge $L_D$ in Umfangrichtung um den Körper (3), wobei der Draht (5) mit einem Lagesensor (4) in derartigem Wirkkontakt steht, daß der Lagesensor (4) ein Signal S ausgibt, sobald der Draht (5) auf den Lagesensor (4) mindestens eine vorbestimmte Zugkraft Z ausübt,

b) Einbringen des losen Endes des Drahtes (5) in eine Antriebseinrichtung (6, 7), die mit einem Wegmesser in Wirkkontakt steht,

c) Spannen des Drahtes (5) entlang des Umfangs (2) des Körpers (3) durch Inbetriebnahme der Antriebseinrichtung (6, 7) bis der mit dem Draht (5) in Wirkkontakt stehende Lagesensor (4) ein Signal gibt,

d) Ablesen des mit dem Wegmesser bestimmten Wegs $W_D$, den der Draht (5) beim Spannen entlang des Umfangs (2) des Körpers (3) durch die Antriebseinrichtung (6, 7) hindurch zurückgelegt hat,

e) Bestimmen des Umfangs (2) des Körpers (3) aus der Länge $L_D$, dem Weg $W_D$ und einem vorrichtungsabhängigen Geometriefaktor G.

8. Verwendung der Vorrichtung nach einem der Ansprüche 1 bis 6 oder des Verfahrens nach Anspruch 7 zur Plethysmographie, insbesondere zur Bestimmung des Umfangs von Extremitäten.

**Claims**

1. A device (1) for measuring the periphery (2) of a body (3), comprising at least the following elements:

(a) a wire (5) having a known longitudinal section of a length $L_D$,

(b) a position sensor (4) so cooperating with wire (5) as to generate a signal S as soon as wire (5) exerts at least a predetermined tractive force Z on position sensor (4),

(c) an actuator (6, 7) for stressing the longitudinal section of length $L_D$ of wire (5) across the periphery (2) of body (3), with the actuator (6, 7) being in communication with the position sensor (4) such that upon generation of signal S the stressing action on wire (5) through actuator (6, 7) can be suspended;

(d) a distance gauge cooperating with actuator (6, 7) for measuring the distance $W_D$ covered by wire (5) during the stressing process along the periphery (2) of body (3) through actuator (6, 7),

(e) so as to determine the periphery (2) of body (3) from the length $L_D$, the distance $W_D$, and a geometric coefficient G dependent on the device, with the device being **characterized in that** the wire (5) comprises a free end and the actuator (6, 7) is able to thread the free end of the wire (5).

2. A device according to claim 1, **characterized in that** it comprises a unit for calculating and sensing the periphery (2) of body (3) from the length $L_D$, the distance $W_D$, and a geometric coefficient G dependent on the device.

3. A device according to any one of the preceding claims, **characterized in that** the actuator (6, 7) forms a calender drive comprising a driving drum (6) and a tractive drum (7).

4. A device according to any one of the preceding claims, **characterized in that** at least a part of the surfaces of the actuator (6, 7) in contact with the wire (5) during its being stressed along the periphery (2) of the body (3) are of a rough structure attainable by a laser treatment.

5. A device according to any one of the preceding claims, **characterized in that** the wire (5) is a nylon filament.

6. A device according to any one of the preceding claims, **characterized in that** the actuator (6, 7) comprises an electromotor.

7. A method for measuring the periphery (2) of a body (3) comprising at least the following steps:

(a) placing a wire (5) including a free end and comprising a known longitudinal section of a length $L_D$, in the circumferential direction, about body (3), with the wire (5) cooperating with a position sensor (4) such that the said position sensor (4) generates a signal S once the wire (5) exerts at least a predetermined tractive force Z on the position sensor (4),

(b) introducing the free end of wire (5) into an actuator (6, 7) cooperating with a distance gauge,

(c) stressing the wire (5) across the periphery (2) of the body (3) by operating the actuator (6, 7) until the position sensor (4) cooperating with the wire (5) generates a signal,

(d) reading the distance $W_D$ measured by the distance gauge that has been covered by the wire (5) during the stressing action across the periphery (2) of the body (3) through the actuator (6, 7),

(e) determining the periphery (2) of the body (3) from the length $L_D$, the distance $W_D$, and a geometric coefficient G dependent on the device.

8. The use of the device according to any one of claims 1 through 6 or according to the method of claim 7 for plethysmographic purposes, in particular, for determining the periphery of extremities.

## Revendications

1. Dispositif (1) pour déterminer le pourtour (2) d'un corps (3), le dispositif (1) présentant au moins les éléments suivants :

   a) un fil (5) avec un segment longitudinal connu d'une longueur $L_D$,

   b) un capteur de position (4), qui est en contact actif avec le fil (5) de telle sorte qu'il émet un signal S dès que le fil (5) exerce au moins une force de traction Z prédéterminée sur le capteur de position (4).

   c) une installation de commande (6, 7) pour tendre le segment longitudinal du fil (5) de la longueur $L_D$ sur le pourtour (2) du corps (3), l'installation de commande (6, 7) étant reliée au capteur de position (4) de telle sorte que lors de l'émission d'un signal S la tension du fil (5) peut être stoppée par l'installation de commande (6, 7),

   d) un capteur de déplacement se trouvant en contact actif avec l'installation de commande (6, 7) pour mesurer la distance du déplacement $W_D$ que le fil (5) parcourt étant tendu sur le pourtour (2) du corps (3) et ayant traversé l'installation de commande (6, 7),

   e) si bien que le pourtour (2) du corps (3) peut être déterminé à partir de la longueur $L_D$, du déplacement $W_D$ et d'un facteur géométrique G dépendant du dispositif,

   et le dispositif étant **caractérisé par le fait que** le fil (5) a une extrémité libre et que l'installation de commande (6, 7) est propre à faire rentrer le bout libre du fil (5).

2. Dispositif selon la revendication 1, **caractérisé en ce que** le dispositif présente une unité pour le calcul et l'émission du pourtour (2) du corps (3) à partir de la longueur $L_D$, du déplacement $W_D$ et d'un facteur géométrique G dépendant du dispositif.

3. Dispositif selon l'une des revendications précédentes, **caractérisé en ce que** l'installation de commande (6, 7) est un engrenage pour calandrer avec un rouleau d'entraînement (6) et un rouleau de traction (7).

4. Dispositif selon l'une des revendications précédentes, **caractérisé par le fait qu'**au moins une partie des surfaces de l'installation de commande (6, 7) qui viennent en contact avec le fil (5) lorsqu'il est tendu sur le pourtour (2) du corps (3), est rugueuse, ce que l'on peut obtenir par traitement au laser.

5. Un dispositif selon l'une des revendications précédentes, **caractérisé en ce que** le fil (5) est un fil de nylon.

6. Un dispositif selon l'une des revendications précédentes, **caractérisé en ce que** l'installation de commande (6, 7) a un moteur électrique.

7. Procédé pour déterminer le pourtour (2) d'un corps (3) présentant au moins les étapes suivantes

   a) Pose d'un fil (5) avec une extrémité libre avec un segment longitudinal connu d'une longueur $L_D$ dans le sens du pourtour autour du corps (3), le fil (5) étant en contact actif avec le capteur de position (4) de telle manière que le capteur de position (4) émet un signal S dès que le fil (5) exerce au moins une force prédéterminée de traction Z sur le capteur de position (4),

   b) entrée de l'extrémité libre du fil (5) dans une installation de commande (6, 7) qui est en contact actif avec un capteur de déplacement,

   c) tension du fil (5) sur le pourtour (2) du corps (3) par la mise en marche de l'installation de commande (6, 7) jusqu'à ce que le capteur de position (4) en contact actif avec le fil (5) donne un signal,

   d) lecture de la distance du déplacement $W_D$, déterminée par le capteur de déplacement, que le fil (5) a parcourue, étant tendu sur le pourtour (2) du corps (3) et ayant traversé l'installation de commande (6, 7),

   e) Détermination du pourtour (2) du corps (3) à partir de la longueur $L_D$, du déplacement $W_D$ et d'un facteur géométrique G dépendant du dispositif.

8. Utilisation du dispositif selon l'une des revendications 1 à 6 ou du procédé selon la revendication 7 se rapportant à la pléthysmographie, en particulier pour déterminer le pourtour d'extrémités.

## FIG. 1